# EUROPEAN PATENT APPLICATION

(11) **EP 3 975 201 A1**
(43) Date of publication of application: **30.03.2022**
(21) Application number: 20290066.8
(22) Date of filing: 28.09.2020
(51) Int. Cl.: G16H 40/60, G06K 9/00

(54) **STERILITY IN AN OPERATION ROOM**

(71) Applicant: Koninklijke Philips N.V., 5656 AG Eindhoven (NL)
(72) Inventor: RONGEN, Peter Maria Johannes, 5656 AE Eindhoven (NL); OLIVAN BESCOS, Javier, 5656 AE Eindhoven (NL); VAN RIEL, Sjors, 5656 AE Eindhoven (NL); DE PAUW, Robert-Jan, 5656 AE Eindhoven (NL); NACHABÉ, Rami, 5656 AE Eindhoven (NL); POST, Wijnand, 5656 AE Eindhoven (NL)
(74) Representative: Philips Intellectual Property & Standards

(57) **Abstract**

The present invention relates to sterility in an operation room. In order to provide improvement for maintaining the sterility, a device (10) for monitoring sterility in an operation room is provided that comprises an image supply (12), a data processor (14) and an output (16). The image supply is configured to provide a stream of image data (18) representing activities during an interventional procedure in the operating room. The data processor is configured to determine at least one sterile zone and at least one non-sterile zone within the operating room based on the stream of image data and to detect activity in the stream of image data that results in sterility breach of the at least one sterile zone and to generate warning data (20) indicating a potential sterility breach. The output is configured to provide the warning data. In an example, a camera grabbing system is provided and various specific smart actions and warnings in the cath lab environment are provided, based on camera observations. The actions are sterility related. The system provides sterility related monitoring without the need for any further input regarding actual scenery data, except for the image stream. In an example, the image feed by the stream of image data is the only required source of information.

## Description

### FIELD OF THE INVENTION

The present invention relates to sterility in an operation room, and relates in particular to a device for monitoring sterility in an operation room, to a system for monitoring sterility in an operation room and to a method for monitoring sterility in an operation room.

### BACKGROUND OF THE INVENTION

During minimal invasive cath lab interventions for treating patients suffering from, for instance, cardio vascular diseases, the clinical staff is using a number of medical equipment and devices like imaging devices such as X-ray, ultrasound etc., disposable intervention tooling, visualization tooling, ambient environment control or the like. A larger part of the equipment and devices is provided in a sterile manner. Because the medical treatment and patient care is of ultimate concern to the clinical staff, any violation of sterility of treatment equipment and tools is preferably undesirable. For example, during endovascular procedures, it is mandatory to preserve the sterility conditions in the cath lab. However, it has been shown that sterility may become broken e.g. due to unexpected user activity.

### SUMMARY OF THE INVENTION

There may thus be a need to provide improvement for maintaining sterility in an operation room.

The object of the present invention is solved by the subject-matter of the independent claims; further embodiments are incorporated in the dependent claims. It should be noted that the following described aspects of the invention apply also for the device for monitoring sterility in an operation room, for the system for monitoring sterility in an operation room and for the method for monitoring sterility in an operation room.

According to the present invention, a device for monitoring sterility in an operation room is provided. The device comprises an image supply, a data processor and an output. The image supply is configured to provide a stream of image data representing activities during an interventional procedure in the operating room. The data processor is configured to determine at least one sterile zone and at least one non-sterile zone within the operating room based on the stream of image data. The data processor is also configured to detect activity in the stream of image data that results in sterility breach of the at least one sterile zone. The data processor is further configured to generate warning data indicating a potential sterility breach. The output is configured to provide the warning data.

As a result, it is provided, for example, to monitor if staff in the non-sterile zone passes non-sterile material to staff in the sterile zone, or if staff in the sterile zone touches any object outside the sterile zone. As an example, this may be caused by stress conditions fatigue, for example caused by too long procedure times, or distractions.

According to an example, the stream of image data comprises at least one of the group of: stream of images from an optical camera; stream of data from depth cameras; and stream of data from a LIDAR sensor providing 3D point clouds.

According to an example, the data processor is trained with basic information to interpret the stream of image data and to determine the sterility and the non-sterility zones.

According to an example, the data processor is configured to provide a self-learning algorithm to be trained with a plurality of image data with identified sterile and non-sterile zones.

According to the present invention, also a system for monitoring sterility in an operation room is provided. The system comprises a device for monitoring sterility in an operation room according to one of the preceding examples and a camera arrangement comprising at least one camera. The camera arrangement is configured to provide the stream of image data.

According to an example, an output arrangement is provided comprising at least one alarm device configured to provide an indication to the staff in the operating room based on the warning data. The indication is selected as at least one from the group of visual indication, acoustic indication and tactile indication.

According to an example, a visualization arrangement is provided that is configured to provide an augmented reality view showing an indication of the sterile zone and the non-sterile zone.

According to the present invention, also a method for monitoring sterility in an operation room is provided. The method comprises the following steps:
- providing a stream of image data representing activities during an interventional procedure in the operating room;
- determining at least one sterile zone and at least one non-sterile zone within the operating room based on the stream of image data;
- detecting activity in the stream of image data that results in sterility breach of the at least one sterile zone;
- generating warning data indicating a potential sterility breach; and
- providing the warning data.

According to an example, based on the detected activity, probabilities for further potential sterility breach are determined and a probability map is generated and provided to the user. In an option provided in addition or alternatively, based on the detected activity, future expected activity is determined and sterility breach of the at least one sterile zone is determined for the future expected activity and a pre-warning data is generated and provided.

According to an example, based on the generated warning data, a probability map of potential infection occurrence is determined and provided to the user.

According to an aspect, a camera grabbing system is provided and various specific smart actions and warnings in the cath lab environment are provided, based on camera observations. The actions are sterility related.

The monitoring of sterility in an operation room is suitable for X-ray catheterization laboratories. The catheterization laboratories are also referred to as cath labs. In an example, the monitoring of sterility is used during a minimal invasive intervention. A system is proposed that warns the staff when it detects a sterility outrage. The basis is the camera and to observe the cath lab situation. Generally, the monitoring of sterility can be used for an application in which cameras detect people and their interaction with environment. The observational system is not necessarily coupled to specific X-ray equipment, but is vendor agnostic.

The system provides sterility related monitoring without the need for any further input regarding actual scenery data, except for the image stream. The image feed by the stream of image data, like the stream of images, is the only required source of information.

In an example, the cameras and the computing unit are provided as separate units. In another example, the cameras and the computing unit are provided in an integrated manner. Thus, intelligent cameras are provided.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for monitoring sterility in an operation room.
Fig. 2 schematically shows an example of a system for monitoring sterility in an operation room.
Fig. 3 shows basic steps of an example of a method for monitoring sterility in an operation room.
Fig. 4 shows an example of a scenery in an operation room in which sterility is monitored.
Fig. 5 shows an example of a camera for observing the operation room, the camera being attached to a display arrangement.
Fig. 6 shows another example of a camera for observing the operation room, the camera being attached to a support mounted in a ceiling corner area of the operation room.
Fig. 7 shows another example of a scenery in an operation room in which sterility is monitored.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

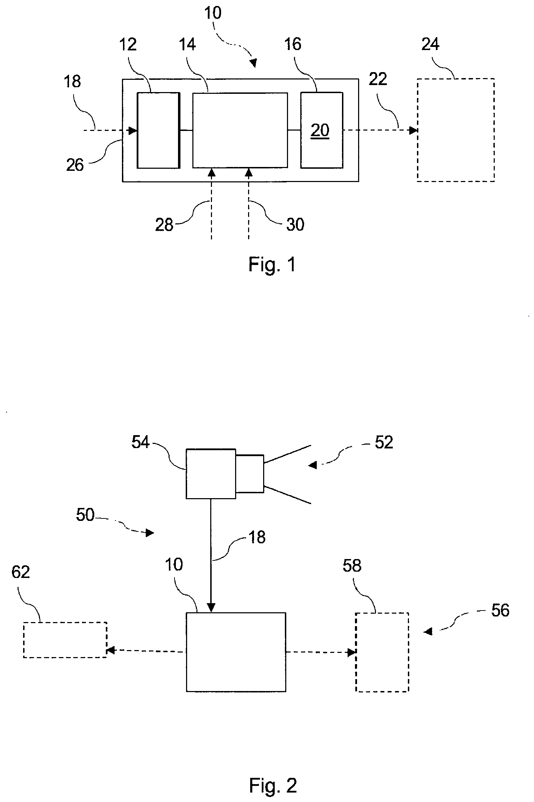

Fig. 1 schematically shows an example of a device 10 for monitoring sterility in an operation room. The device 10 comprises an image supply 12, a data processor 14 and an output 16. The image supply 12 is configured to provide a stream of image data 18 (as indicated with a first hashed arrow) representing activities during an interventional procedure in the operating room. The data processor 14 is configured to determine at least one sterile zone and at least one non-sterile zone within the operating room based on the stream of image data. The data processor 14 is also configured to detect activity in the stream of image data that results in sterility breach of the at least one sterile zone. The data processor 14 is further configured to generate warning data 20 indicating a potential sterility breach. The output 16 is configured to provide the warning data 20. The provision of the warning data 20 is indicated with a second hashed arrow 22.

As an example, the warning data 20 is provided to an indicator arrangement 24, such as a display, for generating a respective visual or acoustic signal to the user, such as staff present in the operation room.

In an example, the image supply 12, the data processor 14 and the output 16 are arranged in a common housing 26. In a further option, the image supply 12, the data processor 14 and the output 16 are arranged as separate components data-connected with each other.

The image supply 12 can also be referred to as image data supply, as image input, as input or as input unit. In an example, the image supply 12 is data-connectable to an imaging source arrangement like a camera. In an example, the image supply 12 is data-connectable to a data storage having stored the sequence of image data. The data processor 14 can also be referred to as data processing arrangement, as processor unit or as processor. In an example, the data processor 14 is data-connected to the image supply 12 and the output 16. The output 16 can also be referred to as output unit. In an example, the output 16 is data-connectable to a warning output arrangement or feedback output arrangement.

The term "zone" relates to a spatial zone, i.e. to a zone within the operation room. The sterile zone may relate to a defined spatial level within the operating room with a defined boundary separating the sterile zone from the non-sterile zone.

The term "operation room" relates to a room or space in a hospital or other medical related building which is provided for examinations, interventions or actual operations. The term comprises any type of room where tasks are performed on or in interaction with a subject, also referred to as patient, and where sterility is required. The term "interventional procedure" thus relates to interventions or actual operations and examinations.

In an example, the stream of image data comprises at least one of the group of: stream of images from an optical camera; stream of data from depth cameras; and stream of data from a LIDAR sensor providing 3D point clouds.

The term "stream of images" relates to a sequence of images, such as a live stream from a camera observing the scenery in the operation room

The term "activities during an interventional procedure" relates to actions and movements by staff members but also actions and movements by the technical equipment used in the operation room.

The term "sterility breach" relates to negatively influencing the degree of sterility in the sterility zone. Examples for sterility breach are contact of sterile parts with non-sterile parts or non-sterile surfaces, or handling of sterile equipment by non-sterile hands of staff members, or depositing used equipment, parts or supply material or consumables or tissue probes in sterile environment.

The sterility zone may be procedure-dependent, e.g. depending on the kind of treatment, the state of the subject, i.e. state of the patient, or other parameters that may have an influence on the sterility and its necessity to provide protection for subject and staff members. For example, the respective required sterility may be defined in dependency of a designated task of the particular device. The sterility may also be defined by sterility classes, e.g. concerning the equipment used. Further, the sterility zone may also be provided in sub-zones with different sterility classes.

The warning data can be used, for example, for generating a warning signal perceptible by at least some of the staff members or by a sterility managing, i.e. sterility responsible staff member. The warning signal can be provided in a manner that it is perceivable and noticeable by the determined person, i.e. intended staff members.

As an example, a cath lab has a sterile zone approximately one meter around the subject, also referred to as patient, on the subject support, also referred to as patient table. The cath lab also has a non-sterile zone, e.g. the rest of the cath lab.

In an example, the data processor 14 comprises an artificial intelligence setup that provides constant self-training.

The data processor 14 is thus provided with basic information only to identify sterile and non-sterile zones.

As an example, feature identification is provided based on image segmentation.

For example, whatever is in blue and/or green in the operation room is considered sterile like the scrubs, the table covers or the bucket containing saline where guide wires and catheters are kept.

In an example, indicated in Fig. 1 as an option, the data processor 14 is trained with basic information 28 (indicated with a third hashed arrow) to interpret the stream of image data and to determine the sterility and the non-sterility zones.

In an example, indicated in Fig. 1 as an alternative or additional option, the data processor 14 is configured to provide a self-learning algorithm to be trained with a plurality of images 30 (indicated with a fourth hashed arrow) with identified sterile and non-sterile zones.

In an example, the data processor 14 comprises a convolutional network configuration. Preferably, the convolutional network configuration is provided with learning images. Further, the convolutional network configuration is configured for a self-learning process to learn the relationship between the first and second images.

In an example, also not further shown in details, the data processor 14 comprises a processing unit operating with classical computer vision.

In another example, also not further shown in details, the data processor 14 comprises a processing unit with artificial intelligence. Artificial intelligence (AI) refers to a computer processor that is configured to execute computational tasks in a smart way. In a certain aspect, artificial intelligence is imitating or emulating ways of human mental processing. In an example, the processing unit with artificial intelligence comprises machine learning or deep learning components. Machine learning (ML) refers to the use of algorithms to analyze and interpret data. The computer learns from analysis and interpretation and uses this for further decisions. Deep learning (DL) refers to the concept that a machine, i.e. a computer processor, is capable of learning to be able to process and solve tasks with increasing complexity. The processor learns and adapts its computational steps by experience. The learning is based on providing artificial neural networks such that increasing data amounts can be processed. For the neural network, the algorithms may be structured in multiple layers.

Fig. 2 schematically shows an example of a system 50 for monitoring sterility in an operation room. The system 50 comprises an example of the device 10 for monitoring sterility in an operation room according to one of the examples described above and below. The system 50 further comprises a camera arrangement 52 comprising at least one camera 54. The camera arrangement 52 is configured to provide the stream of image data 18. The arrow shown in Fig. 2 indicates the data connection. The data connection can be by provided as wireless connection or wire bound connection or other data transfer like optical transfer. In an example, one camera is provided covering the area of an operation table. In another example, several cameras, e.g. at least two cameras, are provided covering the area of the operation table from different viewing angles.

In an example, indicated in Fig. 2 as an option, an output arrangement 56 is provided comprising at least one alarm device 58 configured to provide an indication to the staff in the operating room based on the warning data. The indication is selected as at least one of the group of visual indication, acoustic indication and tactile indication. The hashed arrow shown in Fig. 2 indicates the data connection between the device 10 for monitoring sterility in an operation room and the output arrangement 56. Again, the data connection can be provided as wireless connection or wire bound connection or other data transfer like optical transfer.

The indication may be provided as warning signal informing the user that a current sterility breach is taking place or is about to take place. The visual indication may be provided as a visual warning. The acoustic indication may be provided as an acoustic warning. The tactile indication may be provided as a tactile warning. The visual warning can be provided by additional warning lights or by existing lighting equipment. The visual warning can also be provided by monitors, screens, projections or personal display devices such as augmented reality displays or virtual reality displays. The acoustic warning can be provided by additional acoustic warning components such as loudspeakers or by existing audio equipment. The acoustic warning can also be provided by monitors arrangements with loudspeakers or personal devices such as headphones. The tactile warning can be provided by additional vibration or motion elements or by existing vibration or motion equipment.

In an option, in addition or alternatively, the indication may be provided as learning signal informing the user of sterility issues during an intervention, such as sterility hazards or sterility breaches during the intervention. For example, the indication may be provided as a sterility documentation or sterility information panel, e.g. as a post-interventional sterility dashboard with an overview of potential sterility hazards during the intervention.

In an example, indicated in Fig. 2 as an option, a visualization arrangement 62 is provided that is configured to provide an augmented reality view showing an indication of the sterile zone and the non-sterile zone.

As an example, the indication of the sterile zone and the non-sterile zone is provided overlaid to the stream of images. As another example, the indication of the sterile zone and the non-sterile zone is provided as a projection in the actual live scene.

An example for the visualization arrangement is a display arrangement or personal display devices like head-mounted gear.

In an example, a delineation of the sterile zone versus the non-sterile zone is provided. The augmented reality view on the video stream showing the delineation of sterile versus non-sterile field provides further intuitive information increasing the understanding of the sterility zone.

In an example, not shown in detail, based on the determination of the at least one sterile zone and the at least one non-sterile zone, the at least one camera 54 is configured to adapt at least one of the group of viewing direction, viewing angle and resolution.

The camera 54 can thus zoom-in or zoom-out in dependency of the determined sterility zone. The camera can thus also follow a detected activity to provide a more targeted or more detailed image stream.

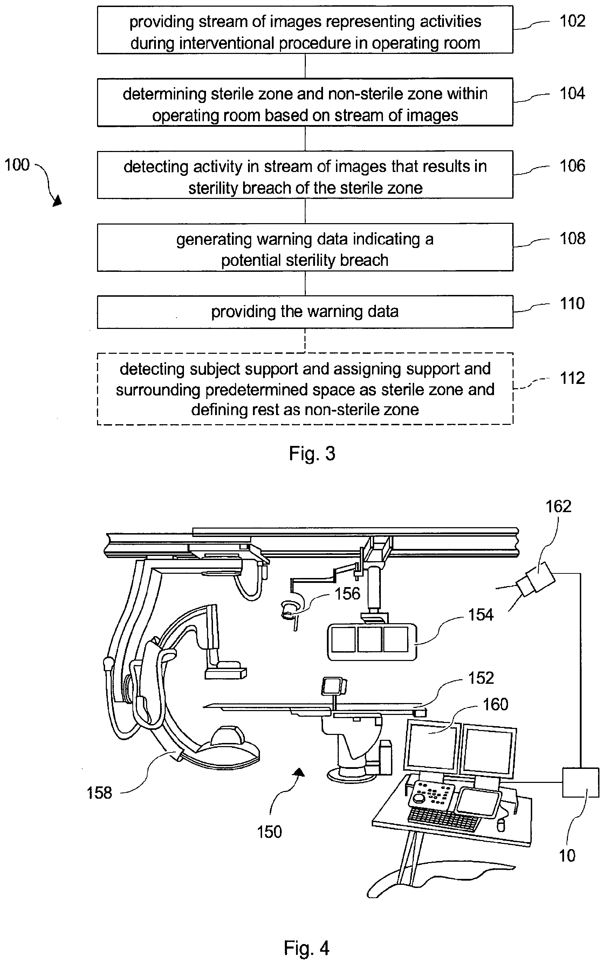

Fig. 3 shows basic steps of an example of a method 100 for monitoring sterility in an operation room. The method 100 comprises the following steps: In a first step 102, a stream of image data representing activities during an interventional procedure in the operating room is provided. In a second step 104, at least one sterile zone and at least one non-sterile zone within the operating room are determined based on the stream of image data. In a third step 106, activity in the stream of image data that results in sterility breach of the at least one sterile zone is detected. In a fourth step 108, warning data is generated indicating a potential sterility breach. In a fifth step 110, the warning data is provided.

In an example, a video grabbing system is provided that records actors, i.e. persons, in the control and intervention room of the cath lab. The data processing unit analyses activity patterns and detects the type of persons and their corresponding sterility classes in the procedure. This processing unit operates with either classical computer vision, or currently state-of-the-art artificial intelligence or deep learning components. As an example, the device observes specific contact situations between persons and objects inside and outside the sterile zone. In case of sterility breach, the device raises a warning to the medical staff.

Examples for breach of sterility comprise at least one of the group of: doctor leaving the sterile zone, touching an object from the non-sterile zone, e.g. a computer mouse, a keyboard or a remote control, and forgetting to swap hand gloves. For example, the doctor grabs a device from a table at the non-sterile zone. For example, the doctor is handed a non-sterile object from a person (not in the sterile zone). For example, the sterile parts (e.g. hands) of the doctor are touched by an object or a person in the non-sterile zone.

In an example, indicated in Fig. 3 as an alternative or additional option, for the determining of the at least one sterile zone and the at least one non-sterile zone, it is provided a sixth step 112 of detecting a subject support in the image data and assigning the subject support and an at least partly surrounding predetermined space around the subject support as the at least one sterile zone enclosed by a sterile boundary and defining the rest of the space inside the operation room as the at least one non-sterile zone.

An example for the surrounding predetermined space is an additional zone of one or two meters or of another practical value.

In another example, not shown in detail, for the detecting of the activity in the stream of image data it is provided at least one of the group of: tracking movements of staff and equipment in relation to the sterile boundary, detecting contact between sterile and non-sterile staff members, detecting contact between sterile and non-sterile equipment, surfaces or supply material, detecting and registering contact between sterile staff members and non-sterile equipment, non-sterile surfaces or non-sterile supply material, and detecting and registering contact between sterile equipment, sterile surfaces or sterile supply material and non-sterile staff members.

For example, it is tracked if a catheter falls on the floor.

In an example, the image data processing comprises segmenting devices in the image stream.

In an example, it is provided the step of detecting individual staff members and their position with respect to the at least one sterile zone and the at least one non-sterile zone.

In an example, for detecting of activity in the stream of image data that results in sterility breach, it is provided the step of detecting non-conformity with predetermined sterility protocols.

An example for a sterility protocol is to take off rings and other cosmetic objections. Another example for a sterility protocol is the obligation to wash hands in advance of a treatment, or to apply disinfectant, or to wear protective gear, i.e. protective clothing and protective equipment.

In another example, not shown in detail, based on the detected activity, probabilities for further potential sterility breach are determined and a probability map is generated and provided to the user. In an option, based on the detected activity, future expected activity is determined and sterility breach of the at least one sterile zone is determined for the future expected activity and a pre-warning data is generated and provided.

The probability map is also referred to as heat map indicating probabilities across the scenery. The probability map provides additional warning data to inform the user where particular care may be appropriate. For determining the further potential sterility breach, detected actions or movements are compared to previous observations and typical occurrences.

The expected activity for the future is based on assumptions that are based on a self-learned algorithm.

In another example, also not shown in detail based on the generated warning data, a probability map of potential infection occurrence is determined and provided to the user.

In an example, a heat map depicting a probabilistic visualization of potential infection occurrence is provided.

For example, in mini-open surgery, the amount of C-arm motion back and forth on top of the surgical field of view is proportional to potential infection rates. Similarly, the amount of times the operation room door is opened and closed can be tracked and used for the heat map. This is provided, for example, for hybrid surgical settings but also for percutaneous procedures.

The probability map of potential infection occurrence provides information for the user where the provision of additional technical measures might be of advantage to minimize sterility breach related risks for subject, i.e. patient, and staff members.

Fig. 4 shows an example of a scenery 150 in an operation room in which sterility is monitored. A subject support 152 is provided. Further, a display arrangement 154 is provided, as well as lighting 156. Both can be movably attached to a ceiling mounted support. Still further, a medical imaging system 158 is shown, e.g. an X-ray imaging system with a movably mounted C-arm carrying an X-ray source and an X-ray detector. A console 160 is indicated for controlling at least a part of the equipment in the operation room. A camera 162 of the camera arrangement 52 is shown. Further, the device 10 for monitoring sterility in an operation room is also indicated connected to the console 160. Connecting lines are indicating the respective data connection. The camera 162 provides the stream of image data 18 representing activities during an interventional procedure in the operating room.

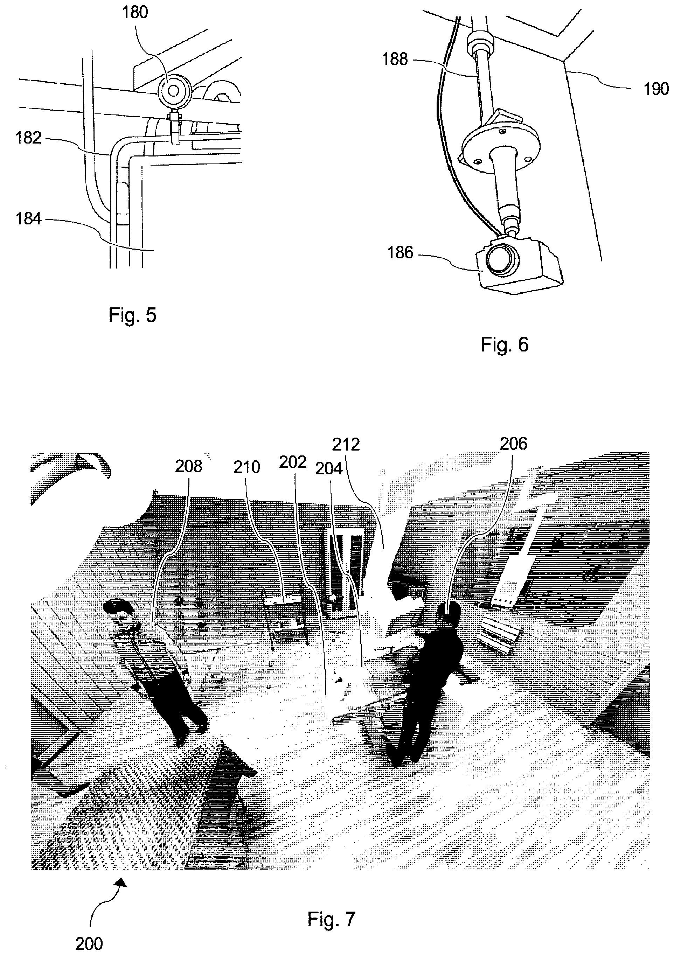

Fig. 5 shows an example 180 of the camera 54 of the camera arrangement 52 for observing the operation room. The camera 54 is attached to a frame structure 182 of a display arrangement 184.

Fig. 6 shows another example 186 of the camera 54 of the camera arrangement 52 for observing the operation room. The camera 54 is attached to a support 188 mounted in a ceiling corner area 190 of the operation room.

Fig. 7 shows another example of a scenery 200 in an operation room in which sterility is monitored. Fig. 7 is shown as seen from a camera mounted in a ceiling area of the operating room. A subject support 202 is provided on which a subject 204 is arranged for an interventional procedure. The subject 204 is partly covered with drapes. A first staff member 206 is standing next to the subject support 202. A second staff member 208 is shown to the left with a distance, for example for arranging and handling equipment like tools or supply material. Several tables 210, movable and non-movable, and other carrier components are provided. Further, also a C-arm X-ray imaging system 212 is shown as an example for imaging equipment used before, during or after an intervention. Sterility is improved due to moitoring sterily breach based on the camera's image supply.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for monitoring sterility in an operation room, comprising:
- an image supply (12);
- a data processor (14); and
- an output (16);
wherein the image supply is configured to provide a stream of image data (18) representing activities during an interventional procedure in the operating room;
wherein the data processor is configured to determine at least one sterile zone and at least one non-sterile zone within the operating room based on the stream of image data; to detect activity in the stream of image data that results in sterility breach of the at least one sterile zone; and to generate warning data (20) indicating a potential sterility breach; and
wherein the output is configured to provide the warning data.

2. Device according to claim 1, wherein the stream of image data comprises at least one of the group of:
- stream of images from an optical camera;
- stream of data from depth cameras; and
- stream of data from a LIDAR sensor providing 3D point clouds.

3. Device according to claim 1 or 2, wherein the data processor is trained with basic information (28) to interpret the stream of image data and to determine the sterility and the non-sterility zones.

4. Device according to claim 1, 2 or 3, wherein the data processor is configured to provide a self-learning algorithm to be trained with a plurality of images (30) with identified sterile and non-sterile zones.

5. Device according to claim 1, 2, 3 or 4, wherein the data processor comprises at least one of the group of:
- a processing unit operating with classical computer vision, and
- a processing unit with artificial intelligence.

6. A system (50) for monitoring sterility in an operation room, comprising:
- a device (10) for monitoring sterility in an operation room according to one of the preceding claims;
- a camera arrangement (52) comprising at least one camera (54);
wherein the camera arrangement is configured to provide the stream of image data.

7. System according to claim 6, wherein an output arrangement is provided comprising at least one alarm device (56) configured to provide an indication to the staff in the operating room based on the warning data; and
wherein the indication is selected as at least one from the group of visual indication, acoustic indication and tactile indication.

8. System according to claim 6 or 7, wherein a visualization arrangement (62) is provided that is configured to provide an augmented reality view showing an indication of the sterile zone and the non-sterile zone.

9. A method (100) for monitoring sterility in an operation room, comprising the following steps:
- providing (102) a stream of image data representing activities during an interventional procedure in the operating room;
- determining (104) at least one sterile zone and at least one non-sterile zone within the operating room based on the stream of image data;
- detecting (106) activity in the stream of image data that results in sterility breach of the at least one sterile zone;
- generating (108) warning data indicating a potential sterility breach; and
- providing (110) the warning data.

10. Method according to claim 9, wherein, for the determining of the at least one sterile zone and the at least one non-sterile zone, it is provided:
- detecting (112) a subject support in the image data and assigning the subject support and an at least partly surrounding predetermined space around the subject support as the at least one sterile zone enclosed by a sterile boundary and defining the rest of the space inside the operation room as the at least one non-sterile zone.

11. Method according to claim 9 or 10, wherein, for the detecting of the activity in the stream of image data, it is provided at least one of the group of:
- tracking movements of staff and equipment in relation to the sterile boundary;
- detecting contact between sterile and non-sterile staff members;
- detecting contact between sterile and non-sterile equipment, surfaces or supply material;
- detecting and registering contact between sterile staff members and non-sterile equipment, non-sterile surfaces or non-sterile supply material; and
- detecting and registering contact between sterile equipment, sterile surfaces or sterile supply material and non-sterile staff members.

12. Method according to claim 9, 10 or 11, wherein, based on the detected activity, probabilities for further potential sterility breach are determined and a probability map is generated and provided to the user; and
wherein based on the detected activity, future expected activity is determined and sterility breach of the at least one sterile zone is determined for the future expected activity and a pre-warning data is generated and provided.

13. Method according to one of the claims 9 to 12, wherein, based on the generated warning data, a probability map of potential infection occurrence is determined and provided to the user.

14. A computer program enabling a processor to carry out the method of one of the claims 9 to 13.

15. A computer readable medium having stored the program element of claim 14.
